(19) Europäisches Patentamt
European Patent Office
Office européen des brevets

(11) EP 0 673 376 B1

(12) **FASCICULE DE BREVET EUROPEEN**

(45) Date de publication et mention de la délivrance du brevet:
**22.05.1996 Bulletin 1996/21**

(21) Numéro de dépôt: **94902003.6**

(22) Date de dépôt: **09.12.1993**

(51) Int Cl.[6]: **C07D 473/16**, C07D 487/04

(86) Numéro de dépôt international:
**PCT/FR93/01211**

(87) Numéro de publication internationale:
**WO 94/13668 (23.06.1994 Gazette 1994/14)**

(54) **COMPOSES BICYCLIQUES DE PYRIMIDINE, LEUR PROCEDE DE PREPARATION ET LES COMPOSITIONS PHARMACEUTIQUES LES RENFERMANT**

BICYCLISCHE PYRIMIDINDERIVATE, VERFAHREN ZU IHRER HERSTELLUNG UND SIE ENTHALTENDE PHARMAZEUTISCHE PRÄPARATE

BICYCLIC PYRIMIDINE COMPOUNDS, METHOD FOR PREPARING SAME AND PHARMACEUTICAL COMPOSITIONS CONTAINING SAID COMPOUNDS

(84) Etats contractants désignés:
**AT BE CH DE DK ES FR GB GR IE IT LI LU NL PT SE**

(30) Priorité: **11.12.1992 FR 9214913**

(43) Date de publication de la demande:
**27.09.1995 Bulletin 1995/39**

(73) Titulaire: **ADIR ET COMPAGNIE**
**F-92415 Courbevoie Cédex (FR)**

(72) Inventeurs:
- **REGNIER, Gilbert**
  **F-92290 Chatenay-Malabry (FR)**
- **DHAINAUT, Alain**
  **F-78400 Chatou (FR)**
- **ATASSI, Ghanem**
  **F-92400 Saint-Cloud (FR)**
- **PIERRE, Alain**
  **F-78160 Marly-le-Roi (FR)**

(74) Mandataire: **Reverbori, Marcelle**
**ADIR**
**1, rue Carle Hébert**
**F-92415 Courbevoie Cédex (FR)**

(56) Documents cités:
**EP-A- 0 495 982**
**EP-A- 0 514 540**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 673 376 B1

Printed by Jouve, 75001 PARIS (FR)

## Description

### Domaine technique

La présente invention a pour objet de nouveaux composés bicycliques de pyrimidine, leur procédé de préparation et les compositions pharmaceutiques les renfermant.

### Technique antérieure

L'état antérieur de la technique dans le domaine pharmaceutique est illustré notamment par les demandes de brevet européen publiées sous les n° 0 495 982 $A_1$ et 0 514 540 $A_1$ qui concernent respectivement des composés de formule :

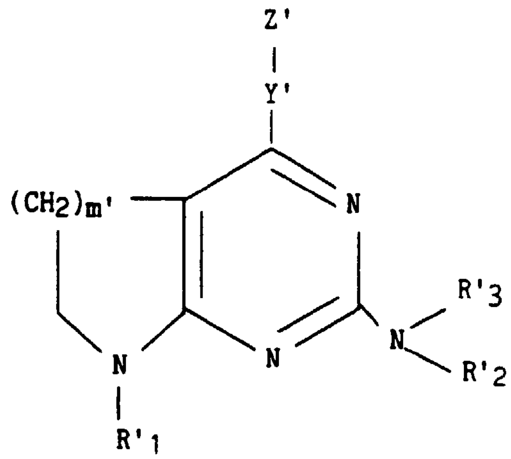

et

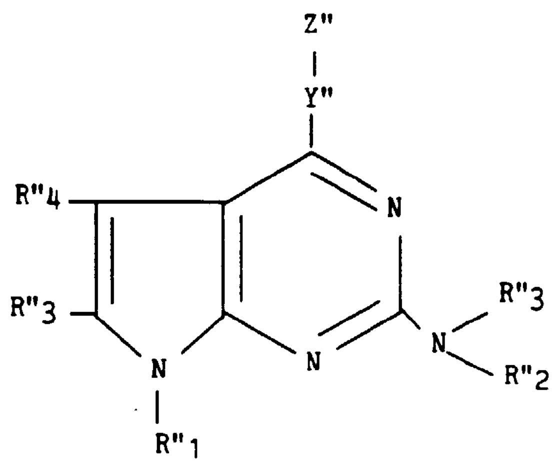

lesquelles formules diffèrent essentiellement des composés de la présente invention par la signification des substituants -Y'-Z' et Y"-Z" sur le noyau pyrimidine, et par leur activité pharmacologique- ces dits produits de l'art antérieur étant actifs contre l'hypoxémie associée aux maladies respiratoires alors que les composés de la présente invention réversent partiellement ou totalement la résistance des cellules tumorales aux agents anti-cancéreux.

### Exposé de l'invention

Elle concerne particulièrement les composés bicycliques de pyrimidine de formule I:

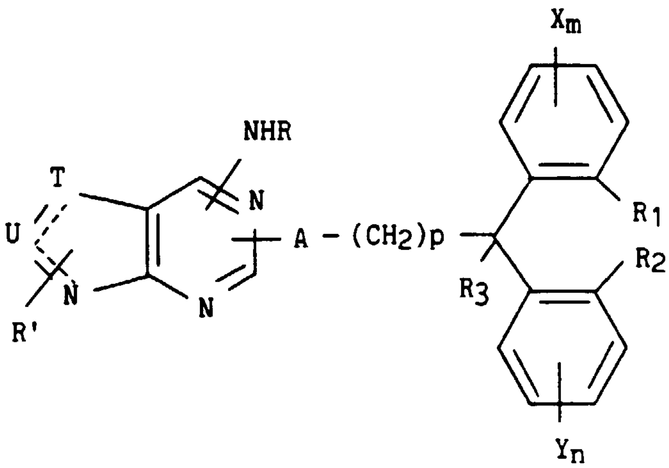

(I)

dans laquelle :

- T et U, identiques ou différents, représentent chacun un radical -CH-ou un atome d'azote de façon à former avec le groupe pyrimidinyle un hétérocycle choisi parmi les : purine, pyrazolo[3,4-d]pyrimidine, pyrrolo[2,3-d]pyrimidine et 1,2,3-triazolo[4,5-d]pyrimidine;
- R et R', identiques ou différents représentent chacun un radical alkyle ayant de 1 à 5 atomes de carbone ou un radical alkényle ayant de 2 à 5 atomes de carbone, chacun en chaîne droite ou ramifiée ;
- A représente un radical hétéromonocyclique de formule :

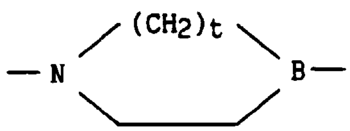

dans laquelle :

- B représente un radical de formule :

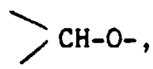 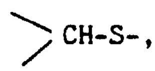 ou 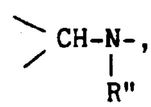

dans lequel R" est un atome d'hydrogène ou un radical alkyle ayant de 1 à 5 atomes de carbone en chaîne droite ou ramifiée et
- t représente un nombre entier de 1 à 3 ;

- p prend les valeurs zéro ou un ;
- X et Y, identiques ou différents, représentent chacun un atome d'hydrogène ou d'halogène ou un radical alkyle ou alkoxy ayant chacun de 1 à 5 atomes de carbone en chaîne droite ou ramifiée ;
- m et n, identiques ou différents, représentent chacun un nombre entier de 1 à 3 ;
- $R_1$ et $R_2$, identiques ou différents, représentent chacun un atome d'hydrogène ou un radical alkyle ayant de 1 à 5 atomes de carbone en chaîne droite ou ramifiée, ou représentent ensemble une liaison simple, un atome d'oxygène ou de soufre, un radical $-(CH_2)r-$ dans lequel r représente un nombre entier de 1 à 3, un radical -CH=CH-, $-O-CH_2-$, $-S-CH_2-$, $-SO_2-CH_2-$, $-CO-CH_2-$,

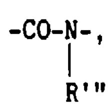

ou

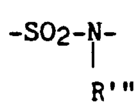

dans lesquelles R'" représente un atome d'hydrogène ou un radical alkyle ayant de 1 à 5 atomes de carbone en chaine droite ou ramifiée, ou $R_1$ et $R_2$ représentent ensemble le radical

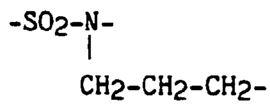

dont le $CH_2$ terminal est relié au carbone le plus proche du cycle benzénique adjacent de façon à ce que l'ensemble :

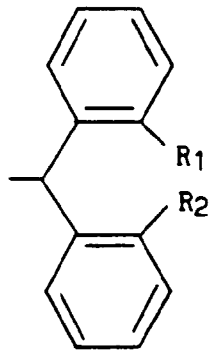

forme le radical tétracyclique de formule :

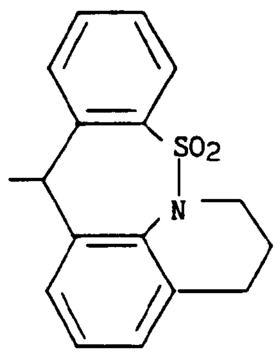

;

et

- $R_3$ représente un atome d'hydrogène ou un radical phényle ;
  ainsi que les composés chiraux et leurs énantiomères.

La présente invention a également pour objet le procédé de préparation des composés de formule I caractérisé en ce que :
l'on condense :

- un composé chloré de formule II :

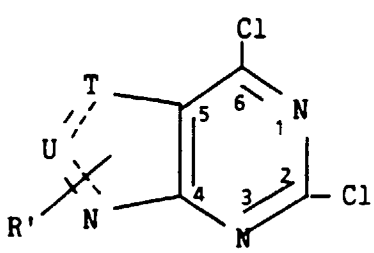

(II)

dans laquelle T, U et R' ont les significations précédemment définies,
avec l'amine de formule :

$$RNH_2 \qquad (III)$$

ou

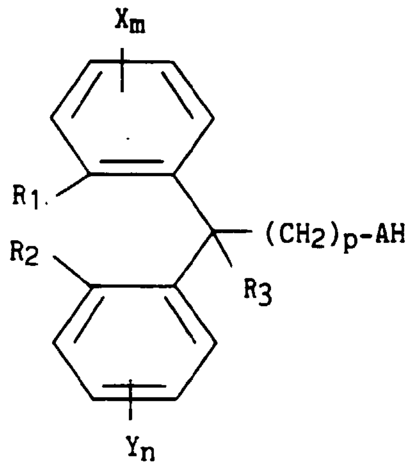

(IV)

que l'on veut fixer en position 6, (R, X, Y, m, n, $R_1$, $R_2$, $R_3$, p et A étant, dans ces formules, tels que précédemment définis)

puis l'on condense le produit ainsi obtenu, selon le cas de formule :

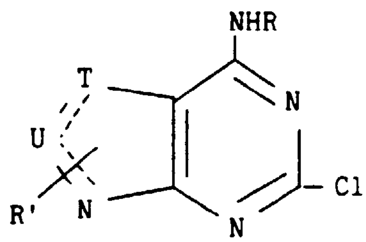

(V)

ou

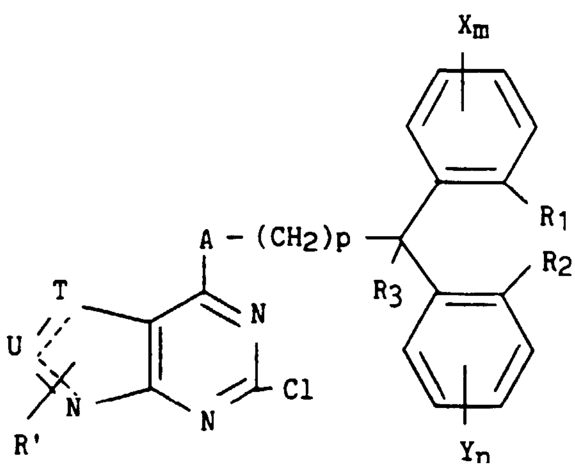

(VI)

avec l'autre amine de formule III ou IV que l'on veut fixer en position 2 -l'ordre de réactivité des amines sur les dérivés dichlorés II étant connu [cf. Heterocyclic compounds Part II, Ed. DJ Brown, Wiley-Interscience (1971) Chapter V, p 160].

Il est particulièrement avantageux de réaliser ces condensations successives comme suit :

La réaction du composé II avec l'amine que l'on veut fixer en position 6 est réalisée dans un solvant polaire tel que, par exemple, un alcool contenant de 2 à 5 atomes de carbone, le diméthylformamide ou le diméthylacétamide à une température voisine de 60 °C, en présence d'un accepteur de l'hydracide formé au cours de la réaction ; cet accepteur pouvant être par exemple, la triéthylamine ou un excès de l'amine utilisée pour la réaction.

Le produit de condensation ainsi obtenu est alors condensé avec l'autre amine (à fixer en position 2) dans un solvant polaire du même type que celui utilisé pour la condensation précédente, en opérant dans un autoclave à une température de 130 à 150 °C, en présence d'un accepteur de l'hydracide formé au cours de la réaction ; cet accepteur étant par exemple la triéthylamine ou un excès de l'amine servant à la réaction.

Les matières premières de formule II ont été préparées à partir de composés dichlorés connus tels que :

- le 2,4-dichloropyrrolo[2,3-d]pyrimidine, (cf ROBINS R.K et coll, JACS (1984) 106, 6379) ;

- le 5,7-dichlorotriazolo[4,5-d]pyrimidine (cf BITTERLI P., ERLENMEYER H., Helv Chem. Acta (1951) 34, 835), et
- le 4,6-dichloropyrazolo[3,4-d]pyrimidine, (cf ROBINS R.K, JACS (1957) 79, 6407).

Ces composés dichlorés qui répondent à la formule :

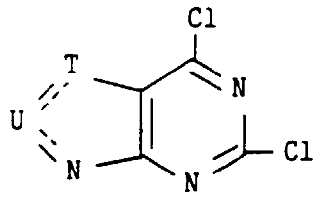

sont alors mis à réagir avec avec un alcool de formule :

R'-OH

dans laquelle R' a la signification précédemment définie, selon la méthode de Mitsunobu -Synthesis (1981), 1, par analogie avec M. IWAKAWA et coll., Can. J. Chem. (1978) 56, 326, et TOYOTA A. et coll., Chem Pharm. Bull. (1992), 40, 1039 -pour donner les matières premières de formule II.

Dans certains cas, on peut obtenir des isomères de substitution que l'on peut séparer par chromatographie.

Les composés de formule I peuvent être transformés en sels d'addition avec les acides, sels qui font, à ce titre, partie de la présente invention. Comme acides utilisables pour la formation de ces sels, on peut citer par exemple, dans la série inorganique, les acides chlorhydrique, bromhydrique, sulfurique, nitrique, phosphorique, et dans la série organique, les acides acétique, propionique, maléique, fumarique, tartrique, oxalique, benzoïque, méthane sulfonique et iséthionique.

Les composés de formule I peuvent être purifiés par des méthodes physiques telles que recristallisation des bases ou des sels, chromatographie (en particulier chromatographie flash sur silice 35-70µ en éluant avec l'acétate d'éthyle ou les systèmes $CH_2Cl_2$-méthanol ou $CH_2Cl_2$-acétone), ou chimiques telles que formation de sels d'addition avec des acides et décomposition de ces sels par des agents alcalins.

Les composés de formule I et leurs sels d'addition physiologiquement tolérables possèdent des propriétés pharmacologiques et thérapeutiques intéressantes et en particulier réversent partiellement ou totalement la résistance des cellules tumorales aux agents anti-cancéreux, ce qui permet de les utiliser comme adjuvants dans le traitement des cancers, dans le but de diminuer ou supprimer la résistance des cellules tumorales aux agents anticancéreux.

La présente invention a également pour objet les compositions pharmaceutiques contenant comme principe actif, un composé de formule I ou un de ses sels physiologiquement tolérable, mélangé ou associé à un excipient pharmaceutique approprié.

Les compositions pharmaceutiques ainsi obtenues se présentent généralement sous forme dosée et peuvent contenir de 0,1 à 1000 mg de principe actif. Elles peuvent revêtir, par exemple, la forme de comprimés, dragées, gélules, suppositoires, solutions injectables ou buvables et être administrées par la voie orale, rectale ou parentérale.

La posologie varie notablement selon l'âge et le poids du patient, la voie d'administration, la nature de la maladie et les traitements associés et s'échelonne de 0,1 à 1000 mg de principe actif par prise.

Les exemples suivants illustrent l'invention. Les points de fusion sont déterminés au tube capillaire (cap) ou à la platine chauffante de Kofler (K).

**Exemple 1**

9-allyl-6-allylamino-2-{4-[(10,11-dihydro-5H-dibenzo[a,d]cycloheptén-5-yl)méthylamino]pipéridino}purine.

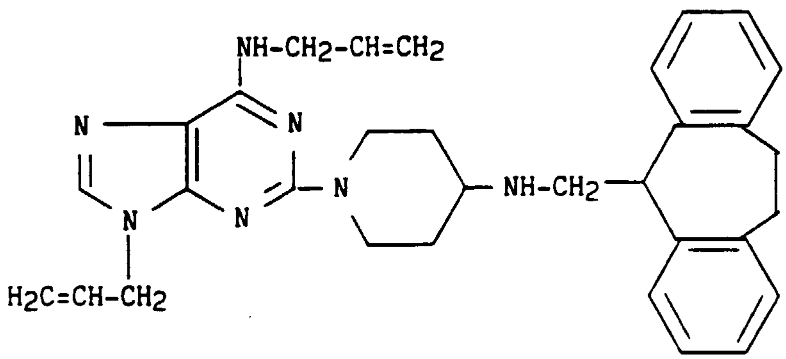

A une solution de 1,15 g de 2,6-dichloro-9-allylpurine dans 15 ml d'éthanol, on ajoute 0,75 ml d'allylamine et agite le mélange pendant 1 heure à température ordinaire, puis on le chauffe à 50 °C pendant 30 minutes. Après évaporation

du solvant, on reprend le mélange avec $CH_2Cl_2$-$H_2O$, décante et évapore le solvant. Le résidu est recristallisé dans l'éthanol.

On obtient 1 g de 2-chloro-6-allylamino-9-allylpurine fondant à 110-112 °C. On dissout 0,85 g de ce composé dans 20 ml de butanol et à la solution obtenue, on ajoute 1,04 g de 4-[(10,11-dihydro-5H-dibenzo [a,d]cycloheptén-5-yl) méthylamino]pipéridine (huile), 0,5 g de $K_2CO_3$ et 0,1 g d'iodure de potassium, et chauffe le mélange à reflux pendant 10 heures.

Ensuite, on concentre le mélange réactionnel et reprend le résidu par $CH_2Cl_2$-$H_2O$.

On décante, puis évapore la phase organique et chromatographie le résidu en éluant avec l'acétate d'éthyle.

On isole 0,6 g de 9-allyl-6-allylamino-2-{4-[(10,11-dihydro-5H-dibenzo [a,d]cycloheptén-5-yl]méthylamino]pipéridino)purine, sous forme de cristaux fondant (cap) à 142-144 °C.

La 9-allyl-2,6-dichloropurine de départ a été préparée en faisant réagir l'alcool allylique et la 2,6-dichloropurine dans le tétrahydrofurane, selon la méthode de Mitsunobu.

L'amine de départ a été préparée par débenzylation de la pipéridine correspondante fondant à 78-81 °C, elle-même préparée par réduction alkylative, avec NaBH3CN dans le méthanol d'un mélange de 1-benzylpipéridone et du chlorhydrate de (10,11-dihydro-5H-dibenzo[a,d]cycloheptén-5-yl)méthylamine fondant (cap) à 275-280 °C.

**Exemple 2**

9-allyl-2-allylamino-6-{4-[(10,11-dihydro-5H-dibenzo[a,d]cycloheptén-5-yl) méthylamino]pipéridino}purine.

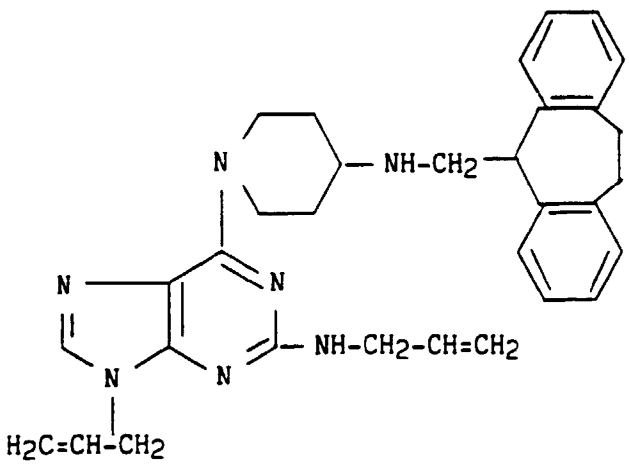

A une solution de 1,15 g de 9-allyl-2,6-dichloropurine dans 20 ml d'éthanol, on ajoute 3 g de 4-[(10,11-dihydro-5H-dibenzo[a,d]cylcloheptén-5-yl)méthylamino]pipéridine et agite le mélange pendant 3 heures à température ambiante, puis pendant 30 minutes à 50 °C.

Le mélange est ensuite traité comme décrit dans l'exemple 1. Le résidu obtenu est recristallisé dans l'éther.

On obtient 1,8 g de cristaux blancs fondant (cap) à 146-148 °C.

En opérant comme dans l'exemple 1, à partir de 1,8 g du dérivé de la 2-chloropurine ainsi obtenu dissout dans 20 ml de butanol, chauffé à 150 °C en présence de 0,6 ml d'allylamine, on obtient 0,7 g de 9-allyl-2-allylamino-6-{4-[(10,11-dihydro-5H-dibenzo[a,d]cycloheptén-5-yl) méthylamino]pipéridino)purine, sous forme de produit résineux dont le difumarate, recristallisé dans l'éthanol fond (cap) à 191-194 °C.

**Exemple 3 à 28**

En appliquant la méthode décrite dans les exemples précédents, ont été préparés les dérivés suivants :

3) 9-allyl-6-allylamino-2-{4-[2,2-bis(4-fluorophényl)éthylamino] pipéridino}purine, PF (cap) : 143-145 °C.

4) 9-allyl-2-allylamino-6-{4-[2,2-bis(4-fluorophény1)éthy1anino] pipéridino)purine, PF (cap) du dimaléate correspondant : 151-152 °C.

5) 9-allyl-6-allylamino-2-{4-[(6,11-dihydrodibenzo[b,e]oxépin-11-yl) méthylamino]pipéridino}purine, P.F (K) du fumarate correspondant : 240 °C.

6) 9-allyl-6-allylamino-2-{4-[(5H-dibenzo[a,d]cycloheptén-5-yl) méthylamino]pipéridino)purine, P.F (K) du fumarate

correspondant : 250 °C.

7) 6-allylamino-2-{4-[(10,11-dihydro-5H-dibenzo[a,d]cycloheptén-5-yl) méthylamino]pipéridino}-9-propylpurine, P. F (K) du fumarate correspondant 237 °C.

8) 9-allyl-2-{4-[(10,11-dihydro-5H-dibenzo[a,d]cycloheptén-5-yl) méthylamino]pipéridino}-6-propylaminopurine, P. F. (K) du fumarate correspondant : 213 °C.

9) 1-allyl-4-allylamino-6-{4-[(10,11-dihydro-5H-dibenzo[a,d]cycloheptén-5-yl)méthylamino]pipéridino}pyrazolo[3,4-d] pyrimidine, P.F (cap) du fumarate correspondant : > 230 °C avec décomposition.

10) 7-allyl-4-allylamino-2-{4-[(10,11-dihydro-5H-dibenzo[a,d]cycloheptén-5-yl)méthylamino]pipéridino}pyrrolo[2,3-d] pyrimidine, P.F (cap) du fumarate correspondant : 160-163 °C.

11) 3-allyl-7-allylamino-5-{4-[(10,11-dihydro-5H-dibenzo[a,d]cycloheptén-5-yl)méthylamino]pipéridino}-1,2,3-triazolo(4,5-d)pyrimidine, P.F. (cap) du fumarate correspondant : 159-162 °C.

12) 9-allyl-6-allylamino-2-{4-[(2,2-diphényléthyl) amino]pipéridino} purine, P.F (K) : 150 °C.

13) 9-allyl-6-allylamino 2-{4-[(3-chloro-6-méthyl-5,5-dioxo-6,11-dihydrodibenzo)[c,f][1,2]thiazépin-11-yl)méthylamino] pipéridino} purine, P.F (K) du fumarate correspondant : 174 °C.

14) 9-allyl-6-allylamino-2-{4-[(2-méthoxy-10,11-dihydro-5H-dibenzo [a,d] cycloheptén-5-yl)méthylamino]pipéridino}purine, P.F (K) du fumarate correspondant : 205 °C.

15) (+) (R) ou (S) 9-allyl-6-allylamino-2-{4-[(3-chloro-6-méthyl-5,5-dioxo-6,11-dihydrodibenzo)[c,f][1,2]thiazépin-11-yl)amino]pipéridino} purine, P.F (K) du dichlorhydrate correspondant : 174 °C.

16) (-) (R) ou (S) 9-allyl-6-allylamino-2-[4-[(3-chloro-6-méthyl-5,5-dioxo-6,11-dihydrodibenzo)[c,f][1,2]thiazépin-11-yl)amino] pipéridino}purine, P.F (K) du dichlorhydrate correspondant : 210 °C.

17) 9-allyl-2-allylamino-6-{4-[(3-chloro-6-méthyl-5,5-dioxo-6,11-dihydrodibenzo)[c,f][1,2]thiazépin-11-yl)méthylamino] pipéridino} purine, P.F (K) du dichlorhydrate correspondant : 210 °C.

18) 9-allyl-2-allylamino-6-{4-[(6,11-dihydro-dibenzo[b,e]oxépin-11-yl)méthylamino]pipéridino} purine, P.F (K) du difumarate correspondant : 186 °C.

19) 9-allyl-2-allylamino-6-{4-[(2-méthoxy-10,11-dihydro-5H-dibenzo[a,d] cycloheptén-yl)méthylamino]pipéridino} purine, P.F (K) du difumarate correspondant : 188 °C.

20) 9-allyl-2-allylamino-6-(4-[(5H-dibenzo[a,d]cycloheptén-5-yl)méthyl amino]pipéridino) purine, P.F (K) du difumarate correspondant : 200 °C.

21) 9-allyl-6-allylamino-2-{4-[(11-méthyl-10,10-dioxo-5,11-dihydro-10-thia-11-aza-dibenzo[a,d]cycloheptén-5-yl) amino]pipéridino} purine, P.F (K) : 182 °C.

22) 9-allyl-6-allylamino-2-{4-[(10,10-dioxo-11-propyl-5,11-dihydro-10-thia-11-aza-dibenzo[a,d]cycloheptén-5-yl) méthylaminompipéridino} purine, P.F (K) : 165 °C.

23) 9-allyl-6-allylamino-2-{4-[(2,3,4-triméthoxy-10,11-dihydro-5H-dibenzo[a,d]cycloheptén-5-yl)méthylamino]pipéridino} purine, P.F (K) du fumarate correspondant : 170 °C.

24) 9-allyl-6-allylamino-2-{4-[(6,11-dihydro dibenzo[b,e]oxépin-11-yl)amino]pipéridino} purine, P.F (K) du fumarate correspondant : 223 °C.

25) 9-allyl-6-allylamino-2-{4-[(5,11-dihydro-6-oxo dibenzo[b,e]azépin-11-yl)méthylamino]pipéridino} purine, P.F (K) du fumarate correspondant : 240 °C.

26) 9-allyl-6-allylamino-2-{4-[(10,11-dihydro-5H-dibenzo[a,d]cycloheptén-5-yl)amino]pipéridino) purine, P.F (K) : 114 °C.

27) 9-allyl-2-allylamino-6-{4-[(10,11-dihydro-5H-dibenzo[a,d]cycloheptén-5-yl)amino]pipéridino} purine, P.F (K) du fumarate correspondant : 170 °C.

28) 9-allyl-6-allylamino-2-{4-[(2,2,2-triphényl éthyl)amino]pipéridino} purine, P.F (K) : 175 °C.

**Exemple 29 : ETUDE PHARMACOLOGIQUE**

La résistance aux agents anticancéreux est un obstacle majeur à l'efficacité des drogues antitumorales. Parmi les différents types de résistance, la "Multidrug Resistance" (MDR) est particulièrement intéressante, puisqu'elle est induite par des composés d'origine naturelle, actifs contre les tumeurs solides (Anthracyclines, Vinca alcaloides, Epipodophyllotoxines par exemple) et qu'elle est très fréquente dans certains cancers (colon par ex.). Les cellules tumorales, lorsqu'elles sont exposées in vitro ou in vivo à l'une de ces drogues, deviennent résistantes, à divers degrés, à l'ensemble de ces composés. Le phénomène de résistance est dû à l'action d'une protéine membranaire inductible, la P-gP 170, dont le rôle est d'augmenter l'efflux du cytotoxique, donc de diminuer sa concentration intracellulaire, d'où la perte de sensibilité de ces cellules à la drogue.

Des médicaments, utilisés dans d'autres pathologies, sont connus pour réverser, partiellement ou totalement, cette résistance (Tsuruo T., Mechanisms of multidrug resistance and implications for therapy. Int. J. Cancer Res., 79, 285-296,1988 ; Rothenberg, M. and Ling V., Multidrug resistance : molecular biology and clinical relevance, J.N.C.I., 81, 907-910, 1989 ; Gottesman M.M. and Pastan, I., Resistance to multiple chemotherapeutic agents in human cancer cells, Trends Pharmacol. Sci, 9, 54-58, 1989 ; Endicott J.A. and Ling V., The biochemistry of P-glycoprotein-mediated multidrug resistance, Annu. Rev. Biochem., 58, 137-171, 1989.).

L'agent modulateur, lorsqu'il est ajouté en même temps que le cytotoxique, diminue, ou supprime totalement la résistance de type MDR. Certains médicaments, comme le vérapamil, l'amiodarone ou la cyclosporine ont été utilisés en clinique pour lever cette résistance, mais leurs propriétés pharmacologiques intrinsèques et leurs toxicités limitent considérablement leur utilisation. D'où l'intérêt de la recherche de composés réversant le phénotype MDR tout en étant dépourvus d'autres propriétés pharmacologiques et de toxicité.

L'étude pharmacologique des composés de la présente invention a consisté tout d'abord en une évaluation in vitro effectuée sur des cellules résistantes.

Le paramètre mesuré est la cytotoxicité de la drogue antitumorale, quantifiée en absence et en présence du composé reversant .

On a également mesuré l'effet des produits sur la concentration intracellulaire du cytotoxique.

En effet, les composés connus pour réverser la MDR agissent en augmentant la concentration intracellulaire en cytotoxique. Cet effet est la conséquence de l'inhibition de l'action de la P-gP 170 responsable de l'efflux de la drogue.

Cette étude a été complétée par une étude in vivo, en utilisant une leucémie murine résistante à la vincristine (P388/VCR) et en associant les produits à la vincristine.

Matériel et méthodes :

1) Activité in vitro

**Cytotoxicité**

Deux lignées cellulaires résistantes ont été utilisées :

- une lignée de poumon de hamster chinois, DC-3F/AD, dont la résistance a été induite par l'actinomycine D. Son facteur de résistance est supérieur à 10 000, c'est donc une lignée extrêmement résistante ;
- et une lignée humaine, issue d'un carcinome épidermoïde de la bouche, KB-A1 dont la résistance a été induite par l'adriamycine. Son facteur de résistance est d'environ 300.

Ces deux lignées sont résistantes également aux Vinca alcaloïdes (Vincristine et Vinblastine).

Les cellules sont cultivées dans du milieu de culture (RPMI 1640) complet contenant 10 % de sérum de veau foetal, 2 mM de glutamine ; 50 unités/ml de pénicilline, 50 ug/ml de streptomycine, 10 mM d'Hepes.

Les cellules sont réparties dans des microplaques et exposées au composé cytotoxique (Actinomycine D ou adriamycine) à 9 concentrations (dilutions sériées de 2 en 2). Les produits testés pour leur capacité à réverser la MDR sont ajoutés en même temps que le cytotoxique.

Les cellules sont ensuite incubées pendant 4 jours. Le nombre de cellules viables est ensuite quantifié par un essai colorimétrique, le Microculture Tétrazolium Assay (Carmichael J., DeGraff W.G., Gazdar A.F., Minna J.D. and Mitchell J.R. Evaluation of a tetrazolium-based semiautomated colorimetric assay : assessment of chemosensitivity testing, Cancer Res, 47, 936-942, 1987.). Les résultats sont exprimés en IC50, concentration en cytotoxique qui inhibe à 50 % la prolifération des cellules traitées par rapport au témoin.

Les résultats sont exprimés en facteur de réversion (FR), avec

$$FR = \frac{\text{IC50 cytotoxique seul}}{\text{IC50 cytotoxique en présence du composé réversant}}$$

**Cytométrie en flux**

Certains composés anticancéreux comme l'adriamycine (ADR) ont la propriété d'être fluorescents après excitation par une source lumineuse de longueur d'onde connue.

Par la mesure de cette fluorescence, il est ainsi possible de mésurer de façon relative la concentration intracellulaire en ADR. La cytométrie en flux (CMF) est un outil de choix pour effectuer ce type de mesure et ainsi déterminer rapidement si certains composés actifs agissent en augmentant la concentration intracellulaire en ADR.

La lignée cellulaire résistante utilisée est KB-A1.

Les cellules $500.10^3$ par ml ont été exposées simultanément à l'ADR à une concentration fixe (50µM) et aux composés testés à différentes concentrations. Après 5 heures d'incubation, l'accumulation de l'ADR intra-cellulaire a été évaluée par CMF. Les analyses ont été réalisées sur un cytomètre en flux ATC3000 (BRUKER-FRANCE) équipé d'un laser argon 2025 (SPECTRA-PHISICS-FRANCE) optimisé à 488 nm pour une puissance de 600 mW. L'analyse de chacun des échantillons a été effectuée sur un total de 10 000 cellules à une vitesse de 1 000 cellules par seconde.

Les résultats ont été collectés sous forme d'histogrammes linéaires de la fluorescence de l'ADR intra-cellulaire.

Expression des résultats : Pour chacun des histogrammes, le canal moyen (MEAN) de fluorescence a été déterminé par le système informatique de l'appareil. Pour toutes les expériences :

- un contrôle négatif (cellules sans ADR) a fixé le seuil d'autofluorescence.
- un contrôle positif (cellules avec ADR) a déterminé la valeur MEAN=MN1.
- les tubes "tests" (cellules avec ADR et avec produit) ont déterminé pour chacun des produits et à chacune des concentrations les valeurs MEAN = MN2.

Les résultats sont exprimés sous forme de variations de la moyenne de fluorescence obtenue pour chacun des tubes "tests" (MN2) par rapport à la moyenne de fluorescence obtenue avec le contrôle positif (MN1) : VAR-MEAN = MN2-MN1. Le paramètre exprimé est donc l'augmentation de la fluorescence de l'ADR en présence des composés testés.

2) Activité in vivo

**Activité antitumorale**

La lignée sensible parentale P388 (leucémie murine) et la sous-lignée résistante à la vincristine, P388/VCR, ont été fournies par le NCI (Frederick, USA). Les cellules tumorales ($10^6$ cellules) ont été inoculées au jour zéro dans la cavité péritonéale à des souris B6D2F1 femelles (Iffa Credo, France) pesant de 18 à 20 g (groupes de 8 à 10 animaux).

Dans le cas d'une administration i.p des produits à tester, les animaux ont reçu aux jours 1, 5 et 9 :

- une administration de produit à tester de la présente invention à raison de 25, 50 ou 75 mg/kg par voie i.p., puis
- 30 à 60 minutes après, une administration de vincristine (pris comme agent anti-tumoral de référence) à raison de 0,50 mg/kg par voie i.p.

Dans le cas d'une administration p.o des produits à tester, les animaux ont reçu aux jours 1, 2, 3, 4 :

- une administration de produit à tester de la présente invention à raison de 100 mg/kg par voie orale, puis
- 30 à 60 minutes après une administration de vincristine à raison de 0,25 mg/kg par voie i.p.

L'activité anti-tumorale est exprimée en :

$$\frac{T}{C}\% = \frac{\text{Temps de survie médian des animaux traités}}{\text{Temps de survie médian des animaux contrôles}} \times 100$$

L'activité du composé réversant est exprimée en T/V :

$$\frac{T}{V} = \frac{\text{Temps de survie médian des animaux traités par l'association VCR + produit réversant}}{\text{Temps de survie médian des animaux traités par la VCR seule}}$$

Résultats :

1) Activité in vitro

Le tableau 1 donne les valeurs des facteurs de réversion obtenus avec les différents composés avec la lignée DC-3F/AD ainsi que l'augmentation de la fluorescence de l'ADR (VAR-MEAN) obtenue avec les différents composés sur la lignée KB-A1. Les produits sont beaucoup plus actifs que les produits de référence.

Le tableau 2 donne les valeurs des facteurs de réversion obtenus avec les différents composés avec la lignée KB-A1. Les composés de l'invention sont très actifs, certains même beaucoup plus actifs que les produits de référence, et réversent totalement la résistance de KB-A1, (lignée cancéreuse humaine) pour l'adriamycine.

2) Activité in vivo

Le tableau 3 montre l'augmentation de l'activité antitumorale in vivo de la vincristine obtenue avec deux composés représentatifs de la présente invention.

Les produits des exemples 1 et 2 de l'invention administrés i.p augmentent considérablement l'activité antitumorale de la vincristine pour une tumeur résistante, la vincristine ayant alors la même activité que pour une tumeur sensible.

Le tableau 4 montre l'activité antitumorale de produits de la présente invention, administrés p.o, sur la lignée P388/VCR.

Les produits de l'invention sont très actifs même lorsqu'ils sont administrés par voie orale. Les produits les plus actifs restaurent totalement l'activité antitumorale de la vincristine.

**ACTIVITE IN VITRO**

Tableau 1

| PRODUITS | FACTEUR DE REVERSION 5μM DC-3F/AD | ACCUMULATION INTRACELLULAIRE EN ADRIAMYCINE VAR-MEAN à 5μM KB-A1 |
|---|---|---|
| PRODUITS DE REFERENCE | | |
| Verapamil | 3 | 13 |
| Cyclosporine A | 4 | 23 |
| PRODUITS DES EXEMPLES | | |
| 1 | 5173 | 110 |
| 2 | 3368 | 111 |

Tableau 2

| PRODUITS | FACTEUR DE REVERSION 5μM KB-A1 |
|---|---|
| PRODUITS DE REFERENCE | |
| Verapamil | 26 |
| Cyclosporine A | 285 |
| PRODUITS DES EXEMPLES | |
| 5 | 572 |
| 9 | 192 |
| 12 | 450 |

Suite du Tableau sur la page suivante

Tableau 2 (suite)

| PRODUITS | FACTEUR DE REVERSION 5μM KB-A1 |
|---|---|
| 13 | 458 |
| 19 | 309 |
| 21 | 330 |
| 23 | 339 |
| 25 | 521 |
| 28 | 464 |

**ACTIVITE IN VIVO**

Tableau 3:

| Augmentation de l'activité antitumorale de la VCR par les produits de la présente invention administrés i.p sur la lignée P388/VCR | | | |
|---|---|---|---|
| **PRODUITS TESTES** | **DOSE (mg/kg) i.p** | **T/C %** | **T/V** |
| Aucun produit (VCR seule) | - | 140 | 1 |
| Exemple 1 | 25 | 215 | 1,54 |
| | 50 | 239 | 1,71 |
| | 75 | 176 | 1,26 |
| Exemple 2 | 25 | 192 | 1,37 |
| | 50 | 224 | 1,60 |
| | 75 | 235 | 1,68 |

Tableau 4:

| Augmentation de l'activité antitumorale de la VCR par les produits de la présente invention administrés, p.o sur la lignée P 388/VCR | | | |
|---|---|---|---|
| **PRODUITS TESTES** | **DOSE (mg/kg) p.o** | **T/C** | **T/V** |
| Aucun produit (VCR seule) | - | 121 | 1 |
| Produits des exemple | | | |
| 1 | 100 | 209 | 1,73 |
| 2 | 100 | 172 | 1,42 |
| 3 | 100 | 203 | 1,68 |
| 5 | 100 | 196 | 1,62 |
| 6 | 100 | 188 | 1,55 |
| 9 | 100 | 161 | 1,33 |
| 12 | 100 | 192 | 1,59 |
| 14 | 100 | 202 | 1,67 |
| 15 | 100 | 157 | 1,30 |

**Revendications**

**1.** les composés bicycliques de pyrimidine de formule I :

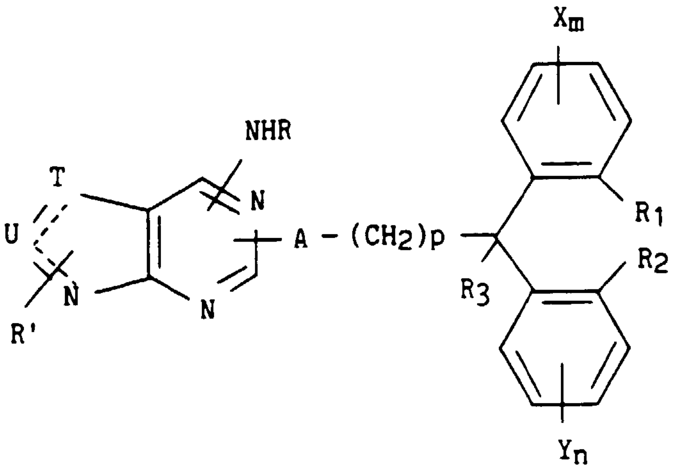

(I)

dans laquelle :

- T et U, identiques ou différents, représentent chacun un radical -CH-ou un atome d'azote de façon à former avec le groupe pyrimidinyle un hétérocycle choisi parmi les : purine, pyrazolo[3,4-d]pyrimidine, pyrrolo[2,3-d] pyrimidine et 1,2,3-triazolo[4,5-d]pyrimidine ;
- R et R', identiques ou différents représentent chacun un radical alkyle ayant de 1 à 5 atomes de carbone ou un radical alkényle ayant de 2 à 5 atomes de carbone, chacun en chaîne droite ou ramifiée ;
- A représente un radical hétéromonocyclique de formule :

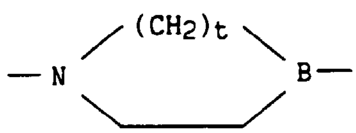

dans laquelle :

- B représente un radical de formule :

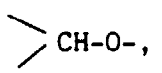
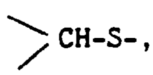 ou 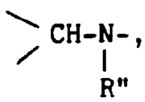

dans lequel R" est un atome d'hydrogène ou un radical alkyle ayant de 1 à 5 atomes de carbone en chaîne droite ou ramifiée et
- t représente un nombre entier de 1 à 3 ;

- p prend les valeurs zéro ou un ;
- X et Y, identiques ou différents, représentent chacun un atome d'hydrogène ou d'halogène ou un radical alkyle ou alkoxy ayant chacun de 1 à 5 atomes de carbone en chaîne droite ou ramifiée ;
- m et n, identiques ou différents, représentent chacun un nombre entier de 1 à 3 ;
- $R_1$ et $R_2$, identiques ou différents, représentent chacun un atome d'hydrogène ou un radical alkyle ayant de 1 à 5 atomes de carbone en chaîne droite ou ramifiée, ou représentent ensemble une liaison simple, un atome d'oxygène ou de soufre, un radical $-(CH_2)r-$ dans lequel r représente un nombre entier de 1 à 3, un radical $-CH=CH-$, $-O-CH_2-$, $-S-CH_2-$, $-SO_2-CH_2-$, $-CO-CH_2-$,

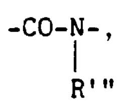

ou

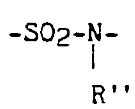

dans lesquelles R'" représente un atome d'hydrogène ou un radical alkyle ayant de 1 à 5 atomes de carbone en chaine droite ou ramifiée, ou $R_1$ et $R_2$ représentent ensemble le radical

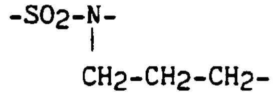

dont le $CH_2$ terminal est relié au carbone le plus proche du cycle benzénique adjacent de façon à ce que l'ensemble :

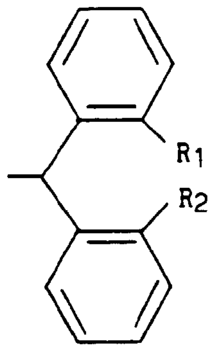

forme le radical tétracyclique de formule :

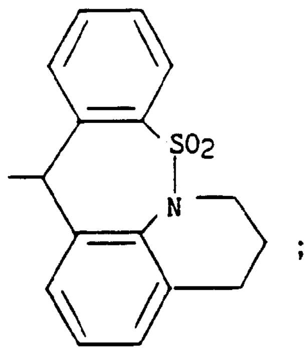

et

- $R_3$ représente un atome d'hydrogène ou un radical phényle ; ainsi que les composés chiraux et leurs énantiomères,

et leurs sels physiologiquement tolérables avec des acides appropriés.

**2.** Un composé de la revendication 1 qui est la 9-allyl-6-allylamino-2-{4-[(10,11-dihydro-5H-dibenzo[a,d]dicycloheptén-5-yl)méthylamino]pipéridino} purine.

**3.** Un composé de la revendication 1 qui est la 9-allyl-6-allylamino-2-{4-[2,2-bis(4-fluorophényl)éthylamino]pipéridino} purine.

**4.** Un composé de la revendication 1 qui est la 9-allyl-6-allylamino-2-{4-[(6,11-dihydrodibenzo[b,e]oxépin-11-yl) méthylamino]pipéridino}purine, et son fumarate correspondant.

**5.** Un composé de la revendication 1 qui est la 9-allyl-6-allylamino-2-{4-[(5H-dibenzo[a,d]cycloheptén-5-yl)méthylamino]pipéridino}purine, et son fumarate correspondant.

**6.** Un composé de la revendication 1 qui est la 1-allyl-4-allylamino-6-{4-[(10,11-dihydro-5H-dibenzo[a,d]cycloheptén-5-yl)méthylamino]pipéridino} pyrazolo[3,4-d]pyrimidine et son fumarate correspondant.

**7.** Un composé de la revendication 1 qui est la 7-allyl-4-allylamino-2-{4-[(10,11-dihydro-5H-dibenzo[a,d]cycloheptén-5-yl)méthylamino]pipéridino} pyrrolo[2,3-d]pyrimidine et son fumarate correspondant.

**8.** Un composé de la revendication 1 qui est la 9-allyl-6-allylamino-2-{4-[(2,2-diphényléthyl)amino]pipéridino}purine.

**9.** Un composé de la revendication 1 qui est la 9-allyl-6-allylamino-2-{4-[(2-méthoxy-10,11-dihydro-5H-dibenzo[a,d] cycloheptén-5-yl)méthylamino] pipéridino}purine et son fumarate correspondant.

**10.** Le procédé de préparation des composés de la revendication 1 caractérisé en ce que : l'on condense :

- un composé chloré de formule II :

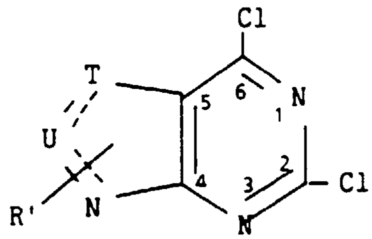

(II)

dans laquelle T, U et R' ont les significations définies dans la revendication 1, avec l'amine de formule :

$$RNH_2 \qquad \text{(III)}$$

ou

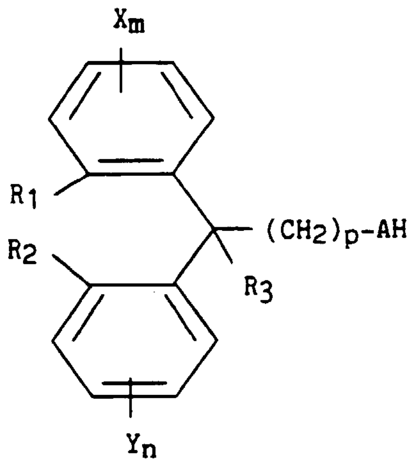

(IV)

que l'on veut fixer en position 6, (R, X, Y, m, n, $R_1$, $R_2$, $R_3$, p et A étant, dans ces formules, tels que définis dans la revendication 1)
puis l'on condense le produit ainsi obtenu, selon le cas de formule :

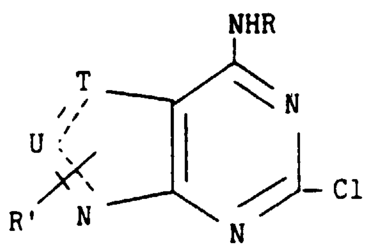

(V)

ou

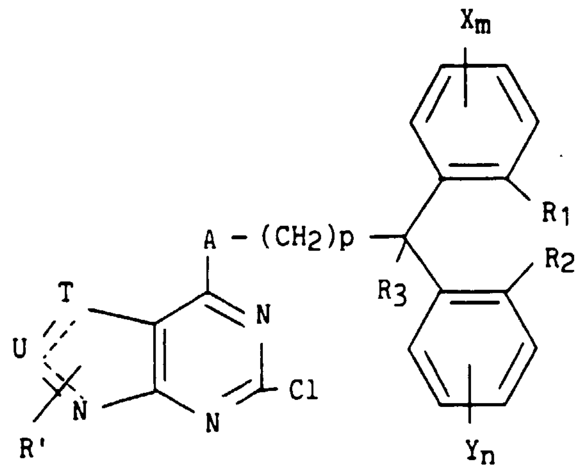

(VI)

avec l'autre amine de formule III ou IV que l'on veut fixer en position 2, (T, U, R, R', A, p, X, Y, m, n, $R_1$, $R_2$ et $R_3$ ayant dans ces formules les significations définies dans la revendication 1).

**11.** Les compositions pharmaceutiques, utilisables pour supprimer la résistance des cellules tumorales aux agents anti-cancéreux, contenant comme principe actif un composé selon l'une quelconque des revendications 1 à 9 avec un ou plusieurs excipients pharmaceutiques appropriés.

**12.** Utilisation d'un composé selon l'une quelconque des revendications 1 à 9 pour la préparation d'un médicament utile pour traiter un mammifère dont l'état nécessite la suppression de la résistance des cellules tumorales aux agents anti-cancéreux.

**Patentansprüche**

**1.** Bicyclische Pyrimidinverbindungen der Formel I:

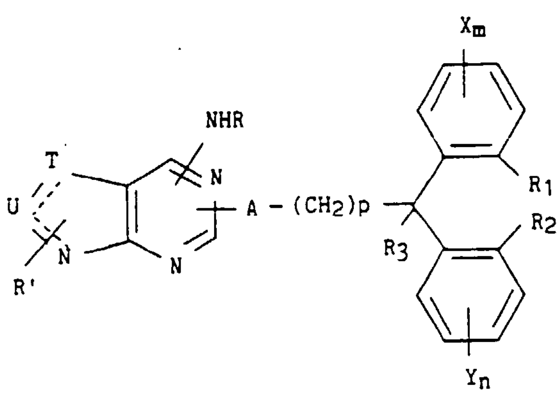

(I)

in der:

- T und U, die gleichartig oder verschieden sein können, jeweils eine Gruppe -CH- oder ein Stickstoffatom bedeuten, so daß sie mit der Pyrimidinylgruppe einen Heterocyclus ausgewählt aus: Purin, Pyrazolo[3,4-d] pyrimidin. Pyrrolo-[2,3-d]pyrimidin und 1,2,3-Triazolo[4,5-d]pyrimidin bilden;
- R und R', die gleichartig oder verschieden sein können, jeweils eine Alkylgruppe mit 1 bis 5 Kohlenstoffatomen oder eine Alkenylgruppe mit 2 bis 5 Kohlenstoffatomen jeweils in gerader oder verzweigter Kette bedeuten;
- A eine heteromonocyclische Gruppe der Formel:

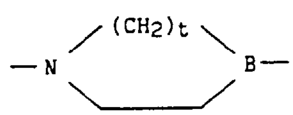

bedeutet, in der:

- B eine Gruppe der Formel:

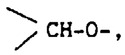 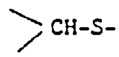 oder 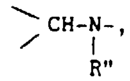

worin R" ein Wasserstoffatom oder eine Alkylgruppe mit 1 bis 5 Kohlenstoffatomen in gerader oder verzweigter Kette ist, und

- t eine ganze Zahl mit einem Wert von 1 bis 3 darstellen;

- p Werte von Null oder Eins aufweist;
- X und Y, die gleichartig oder verschieden sein können, jeweils ein Wasserstoffatom, ein Halogenatom oder eine Alkyl- oder Alkoxy-gruppe mit jeweils 1 bis 5 1 Kohlenstoffatomen in gerader oder verzweigter Kette bedeuten;
- m und n, die gleichartig oder verschieden sein können,jeweils eine ganze Zahl mit einem Wert von 1 bis 3 bedeuten;
- $R_1$ und $R_2$, die gleichartig oder verschieden sein können, jeweils ein Wasser-stoffatom oder eine Alkylgruppe mit 1 bis 5 Kohlenstoffatomen in gerader oder verzweigter Kette oder gemeinsam eine Einfachbindung, ein Sauerstoff- oder Schwefelatom, eine Gruppe $-(CH_2)_r-$, worin r eine ganze Zahl mit einem Wert von 1 bis 3 darstellt, eine Gruppe -CH=CH-, $-O-CH_2-$, $-S-CH_2-$, $-SO_2-CH_2-$, $-CO-CH_2-$,

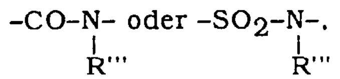

worin R''' ein Wasserstoffatom oder eine Alkylgruppe mit 1 bis 5 Kohlenstoffatomen in gerader oder verzweigter Kette darstellt, oder

$R_1$ und $R_2$ gemeinsam die Gruppe

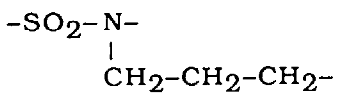

deren endständige $CH_2$-Gruppe mit dem nächsten Kohlenstoffatom des benachbarten Benzolrings verbunden ist, so daß die Gruppe:

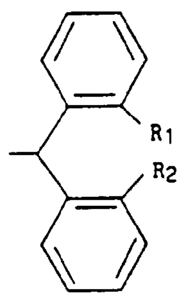

den tetracyclischen Rest der Formel bildet:

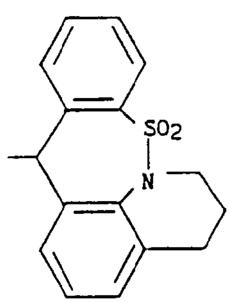

bedeuten; und

- $R_3$ ein Wasserstoffatom oder eine Phenylgruppe bedeutet;

sowie die chiralen Verbindungen und ihre Enantiomere und ihre physiologisch verträglichen Salze mit geeigneten Säuren.

2. Verbindung nach Anspruch 1, nämlich 9-Allyl-6-allylamino-2-{4-[(10,11-dihydro-5H-dibenzo[a,d]dicyclohepten-5-yl)-methylamino]-piperidino}-purin.

**3.** Verbindung nach Anspruch 1, nämlich 9-Allyl-6-allylamino-2-{4-[2,2-bis(4-fluorphenyl]-ethylamino]-piperidino}-purin.

**4.** Verbindung nach Anspruch 1, nämlich 9-Allyl-6-allylamino-2-{4-[(6,11-dihydrodibenzo[b,e]oxepin- 1-yl)-methylamino]-piperidino}-purin und sein entsprechendes Fumarat.

**5.** Verbindung nach Anspruch 1, nämlich 9-Allyl-6-allylamino-2-{4-[(5H-dibenzo[a,d]cyclohepten-5-yl)-methylamino]-piperidino}-purin und sein entsprechendes Fumarat.

**6.** Verbindung nach Anspruch 1, nämlich 1-Allyl-4-allylamino-6-{4-[(10,11-dihydro-5H-dibenzo[a,d]cyclohepten-5-yl)-methylaminol]-piperidino}-pyrazolo-[3,4-d]pyrimidin und sein entsprechendes Fumarat.

**7.** Verbindung nach Anspruch 1, nämlich 7-Allyl-4-allylamino-2-{4-[(10,11-dihydro-5H-dibenzo[a,d]cyclohepten-5-yl)-methylamino]-piperidino}-pyrrolo-[2,3-d]pyrimidin und sein entsprechendes Fumarat.

**8.** Verbindung nach Anspruch 1, nämlich 9-Allyl-6-allylamino-2-{4-[(2,2-diphenylethyl)-amino]-piperidino}-purin.

**9.** Verbindung nach Anspruch 1, nämlich 9-Allyl-6-allylamino-2-{4-[(2-methoxy-10,11-dihydro-5H-dibenzo[a,d]cyclohepten-5-yl)-methylamino]-piperidino}-purin und sein entsprechendes Fumarat.

**10.** Verfahren zur Herstellung der Verbindungen nach Anspruch 1, **dadurch gekennzeichnet,** daß man:

- eine Chlorverbindung der Formel II:

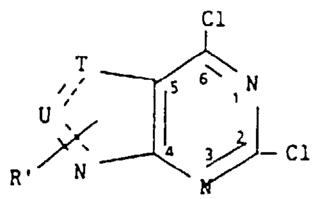

(II)

in der T, U und R' die in Anspruch 1 angegebenen Bedeutungen besitzen, mit dem Amin der Formel:

$$RNH_2 \quad \text{(III)}$$

oder

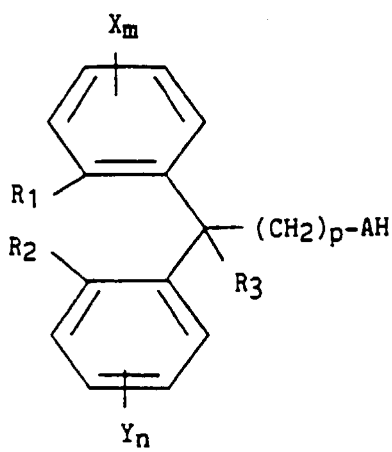

(IV)

welches man in der 6-Stellung binden will (wobei in diesen Formeln R, X, Y, m, n, $R_1$, $R_2$, $R_3$, p und A die in Anspruch 1 angegebenen Bedeutungen besitzen), kondensiert und dann das je nachdem erhaltene Produkt der Formel:

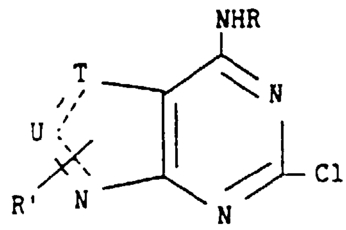

(V)

oder

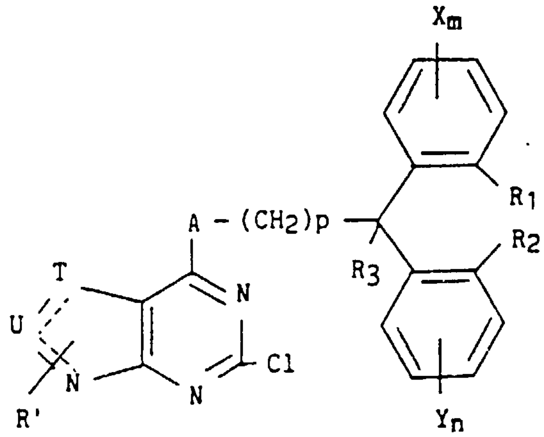

(VI)

mit dem anderen Amin der Formel III oder IV, welches man in der 2-Stellung binden will (wobei in diesen Formeln T, U, R, R', A, p, X, Y, m, n, $R_1$, $R_2$ und $R_3$ die in Anspruch 1 angegebenen Bedeutungen besitzen), kondensiert.

**11.** Pharmazeutische Zubereitungen zur Unterdrückung der Resistenz von Tumorzellen gegen Antikrebsmittel, enthaltend als Wirkstoff eine Verbindung nach einem der Ansprüche 1 bis 9 zusammen mit einem oder mehreren geeigneten pharmazeutischen Trägermaterialien.

**12.** Verwendung einer Verbindung nach einem der Ansprüche 1 bis 9 für die Herstellung eines Arzneimittels zur Behandlung eines Säugers, dessen Zustand die Unterdrückung der Resistenz von Tumorzellen gegen Antikrebsmittel erforderlich macht.

## Claims

**1.** Bicyclic pyrimidine compounds of formula I :

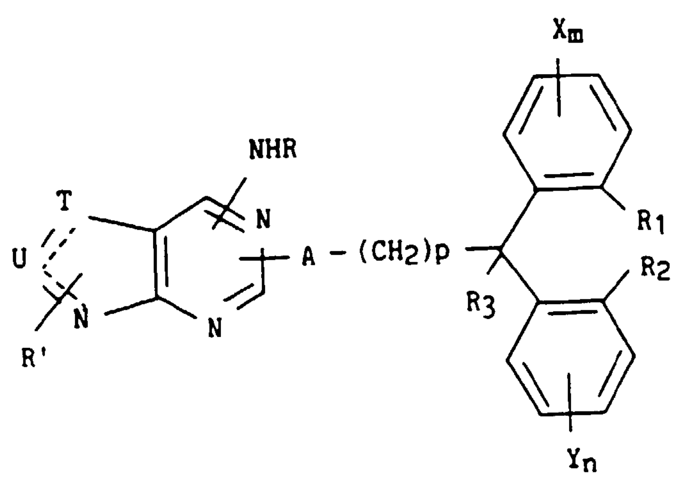

(I)

wherein :

- T and U, which are the same or different, each represents a -CH- radical or a nitrogen atom so as to form with

the pyrimidinyl group a heterocycle selected from: purine, pyrazolo[3,4-d]pyrimidine, pyrrolo[2,3-d]pyrimidine and 1,2,3-triazolo[4,5-d]pyrimidine;

- R and R', which are the same or different, each represents an alkyl radical having from 1 to 5 carbon atoms or an alkenyl radical having from 2 to 5 carbon atoms, each in straight or branched chain;
- A represents a heteromonocyclic radical of formula:

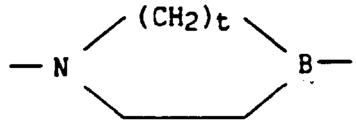

wherein :

- B represents a radical of formula :

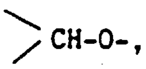 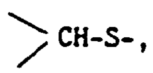 or 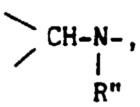

  wherein R" is a hydrogen atom or an alkyl radical having from 1 to 5 carbon atoms in straight or branched chain and
- t represents an integer of from 1 to 3;

- p is zero or one;
- X and Y, which are the same or different, each represents a hydrogen atom, a halogen atom, or an alkyl or alkoxy radical each having from 1 to 5 carbon atoms in straight or branched chain;
- m and n, which are the same or different, each represents an integer of from 1 to 3;
- $R_1$ and $R_2$, which are the same or different, each represents a hydrogen atom or an alkyl radical having from 1 to 5 carbon atoms in straight or branched chain, or together represent a single bond, an oxygen or sulphur atom, a radical $-(CH_2)_r-$ in which r represents an integer of from 1 to 3, a radical -CH=CH-, $-O-CH_2-$, $-S-CH_2-$, $-SO_2-CH_2-$, $-CO-CH_2-$,

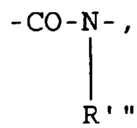

or

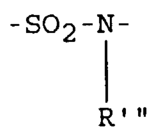

in each of which R''' represents a hydrogen atom or an alkyl radical having from 1 to 5 carbon atoms in straight or branched chain, or

$R_1$ and $R_2$ together represent the radical

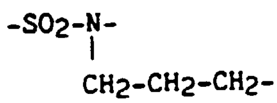

in which the terminal $CH_2$ is attached to the closest carbon of the adjoining benzene ring so that the whole :

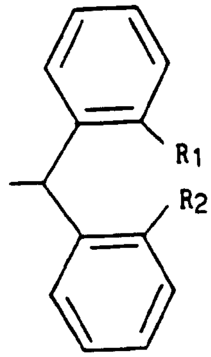

forms the tetracyclic radical of formula :

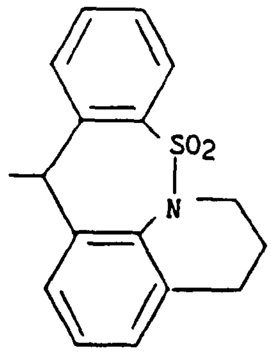

;

and

- $R_3$ represents a hydrogen atom or a phenyl radical;

and also the chiral compounds and their enantiomers, and their physiologically tolerable salts with appropriate acids.

2. A compound of claim 1, which is 9-allyl-6-allylamino-2-{4-[(1ogll-dihydro-5H-dibenzo[a,d]dicyclohepten-5-yl)-methylamino)piperidino}purine.

3. A compound of claim 1, which is 9-allyl-6-allylamino-2-{4-[2,2-bis(4-fluorophenyl)ethylamino]piperidino}-purine.

4. A compound of claim 1, which is 9-allyl-6-allylamino-2-(4-[(6,11-dihydrodibenzo[b,e]oxepin-11-yl)methylamino]-piperidino}purine, and its corresponding fumarate.

5. A compound of claim 1, which is 9-allyl-6-allylamino-2-(4-[(5H-dibenzo[a,d]cyclohepten-5-yl)methylamino]piperidino}purine, and its corresponding fumarate.

6. A compound of claim 1, which is 1-allyl-4-allylamino-6-(4-[(10,11-dihydro-5H-dibenzo[a,d]cyclohepten-5-yl)-methylamino]piperidino}pyrazolo[3,4-d]pyrimidine, and its corresponding fumarate.

7. A compound of claim 1, which is 7-allyl-4-allylamino-2-{4-[(10,11-dihydro-5H-dibenzo[a,d]cyclohepten-5-yl)-methylamino]piperidino}pyrrolo[2,3-d]pyrimidine, and its corresponding fumarate.

8. A compound of claim 1, which is 9-allyl-6-allylamino-2-{4-[(2,2-diphenylethyl)amino]piperidino}purine.

9. A compound of claim 1, which is 9-allyl-6-allylamino-2-{4-[(2-methoxy-10,11-dihydro-5H-dibenzo[a,d]cyclohepten-5-yl)methylamino]piperidino}purine, and its corresponding fumarate.

10. A process for the preparation of compounds of claim 1, characterised in that :

- a chlorinated compound of formula II :

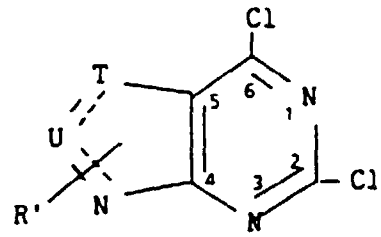

(II)

wherein T, U and R' are as defined in claim 1, is condensed with the amine of formula :

$$RNH_2 \qquad \text{(III)}$$

or

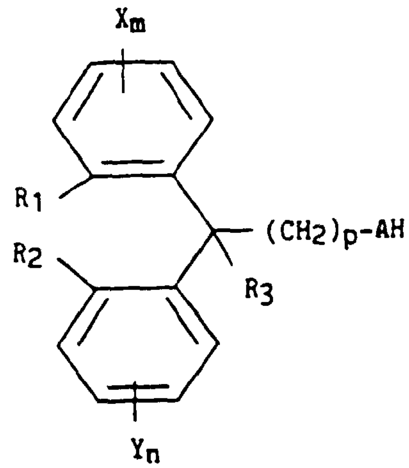

(IV)

whichever it is desired to attach in the 6-position, (R, X, Y, m, n, $R_1$, $R_2$, $R_3$, p and A in those formulae being as defined in claim 1),

then the product so obtained which, depending on the case concerned, is of formula :

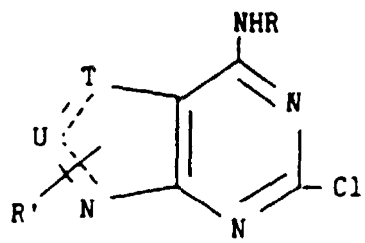

(V)

or

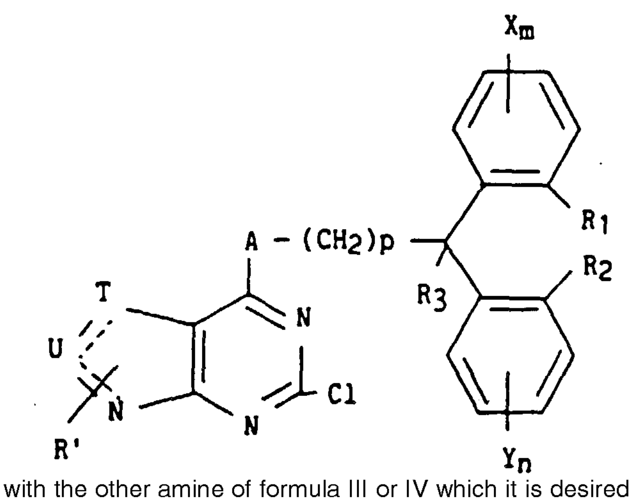

(VI)

is condensed with the other amine of formula III or IV which it is desired to attach in the 2-position (T, U, R,

R', A, p, X, Y, m, n, $R_1$, $R_2$ and $R_3$ in those formulae being as defined in claim 1).

**11.** Pharmaceutical compositions that can be used to suppress the resistance of tumour cells to anti-cancer agents, comprising as active ingredient a compound according to any one of claims 1 to 9 together with one or more appropriate pharmaceutical excipients.

**12.** Use of a compound according to any one of claims 1 to 9 for the preparation of a medicament useful in the treatment of a mammal the condition of which necessitates the suppression of the resistance of tumour cells to anti-cancer agents.